# EUROPEAN PATENT APPLICATION

(11) **EP 4 335 845 A1**
(43) Date of publication of application: **13.03.2024**
(21) Application number: 22873131.1
(22) Date of filing: 06.09.2022
(51) Int. Cl.: C07D 403/10, H10K 99/00, H10K 50/00

(54) **NOVEL COMPOUND AND ORGANIC LIGHT EMITTING DEVICE COMPRISING THE SAME**

(30) Priority: 27.09.2021 KR 20210127023
(71) Applicant: LG Chem, Ltd., Seoul 07336 (KR)
(72) Inventor: HEO, Dong Uk, Daejeon 34122 (KR); YOON, Jung Min, Daejeon 34122 (KR); HAN, Miyeon, Daejeon 34122 (KR); LEE, Jae Tak, Daejeon 34122 (KR); LEE, Jaegoo, Daejeon 34122 (KR); HONG, Sung Kil, Daejeon 34122 (KR); YUN, Heekyung, Daejeon 34122 (KR)
(74) Representative: Hoffmann Eitle
(86) International application number: PCT/KR2022/013337
(87) International publication number: WO 2023/048426

(57) **Abstract**

The present disclosure relates to a novel compound and an organic light emitting device including the same.

## Description

### [TECHNICAL FIELD]

### Cross-reference to Related Application(s)

This application claims the benefit of Korean Patent Application No. 1 0-2021-0127023 filed on September 27, 2021 in the Korean Intellectual Property Office, the disclosures of which are incorporated herein by reference in their entirety.

The present disclosure relates to a novel compound and an organic light emitting device including the same.

### [BACKGROUND OF ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of new materials for the organic materials used in the organic light emitting devices as described above.

### [PRIOR ART LITERATURE]

### [Patent Literature]

(Patent Literature 0001) Korean Unexamined Patent Publication No. 10-2000-0051826

### [DETAILED DESCRIPTION OF THE INVENTION]

### [Technical Problem]

The present disclosure relates to a novel compound and an organic light emitting device including the same.

### [Technical Solution]

In the present disclosure, there is provided a compound represented by the following Chemical Formula 1: 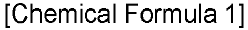 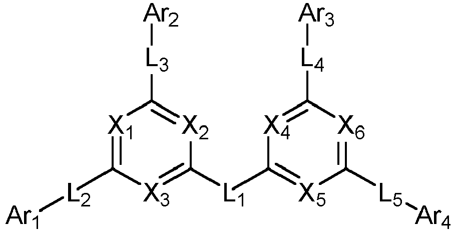 in the Chemical Formula 1,
X₁ to X₆ are each independently N or CH, wherein at least two of X₁ to X₆ are N,
L₁ is represented by the following Chemical Formula 2,
L₂ to L₅ are each independently a single bond, or substituted or unsubstituted C₆₋₆₀ arylene,
Ar₁ to Ar₄ are each independently substituted or unsubstituted C₆₋₆₀ aryl, and
at least one of Ar₁ to Ar₄ is substituted with cyano, 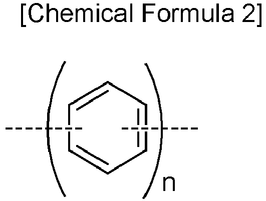
in the Chemical Formula 2,
n is an integer of 3 to 5.

In addition, there is provided an organic light emitting device including: a first electrode; a second electrode that is provided opposite to the first electrode; and one or more organic material layers that are provided between the first electrode and the second electrode, wherein at least one layer of the organic material layers includes the compound represented by the Chemical Formula 1.

### [ADVANTAGEOUS EFFECTS]

The compound represented by the Chemical Formula 1 may be used as a material for an organic material layer of an organic light emitting device, and may improve efficiency, low driving voltage, and/or lifespan of the organic light emitting device. In particular, the compound represented by the Chemical Formula 1 may be used as a material for hole injection, hole transport, hole injection and transport, light emission, electron transport, or electron injection.

### [BRIEF DESCRIPTION OF THE DRAWINGS]

FIG. 1 shows an example of an organic light emitting device including a substrate 1, an anode 2, a light emitting layer 3, and a cathode 4.
FIG. 2 shows an example of an organic light emitting device including a substrate 1, an anode 2, a hole injection layer 5, a hole transport layer 6, a light emitting layer 3, an electron injection and transport layer 7, and a cathode 4.

### [DETAILED DESCRIPTION OF THE EMBODIMENTS]

Hereinafter, embodiments of the present disclosure will be described in more detail to facilitate understanding of the invention.

As used herein, the notation 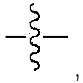 or 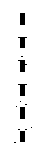 means a bond linked to another substituent group.

As used herein, the term "substituted or unsubstituted" means being unsubstituted or substituted with one or more substituents selected from the group consisting of deuterium; a halogen group; a nitrile group; a nitro group; a hydroxyl group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O and S atoms, or being unsubstituted or substituted with a substituent in which two or more substituents of the above-exemplified substituents are connected. For example, "a substituent in which two or more substituents are connected" may be a biphenyl group. Namely, a biphenyl group may be an aryl group, or it may also be interpreted as a substituent in which two phenyl groups are connected.

In the present disclosure, the carbon number of a carbonyl group is not particularly limited, but is preferably 1 to 40. Specifically, the carbonyl group may be a compound having the following structural formulae, but is not limited thereto.

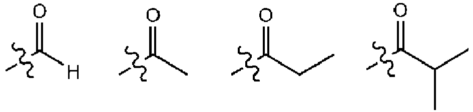

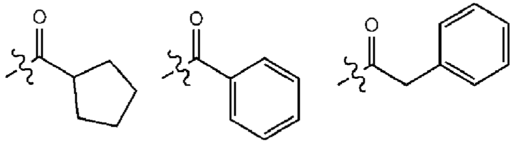

In the present disclosure, an ester group may have a structure in which oxygen of the ester group is substituted by a straight-chain, branched-chain, or cyclic alkyl group having 1 to 25 carbon atoms, or an aryl group having 6 to 25 carbon atoms. Specifically, the ester group may be a compound having the following structural formulae, but is not limited thereto.

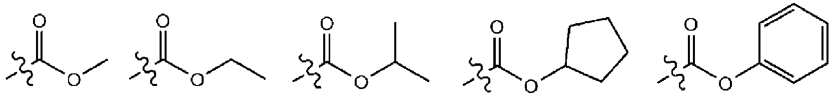

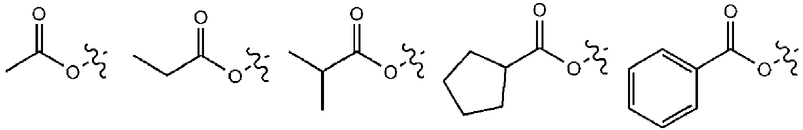

In the present disclosure, the carbon number of an imide group is not particularly limited, but is preferably 1 to 25. Specifically, the imide group may be a compound having the following structural formulae, but is not limited thereto.

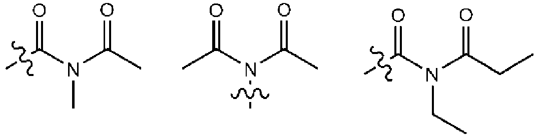

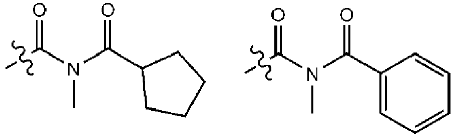

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present disclosure, a boron group specifically includes a trimethylboron group, a triethylboron group, a t-butyldimethylboron group, a triphenylboron group, a phenylboron group, and the like, but is not limited thereto.

In the present disclosure, examples of a halogen group include fluorine, chlorine, bromine, or iodine.

In the present disclosure, the alkyl group may be straight-chain, or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethylpropyl, isohexyl, 2-methylpentyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The monocyclic aryl group includes a phenyl group, a biphenyl group, a terphenyl group and the like, but is not limited thereto. The polycyclic aryl group includes a naphthyl group, an anthracenyl group, a phenanthryl group, a pyrenyl group, a perylenyl group, a chrysenyl group, a fluorenyl group or the like, but is not limited thereto.

In the present disclosure, a fluorenyl group may be substituted, and two substituents may be bonded to each other to form a spiro structure. In the case where the fluorenyl group is substituted, 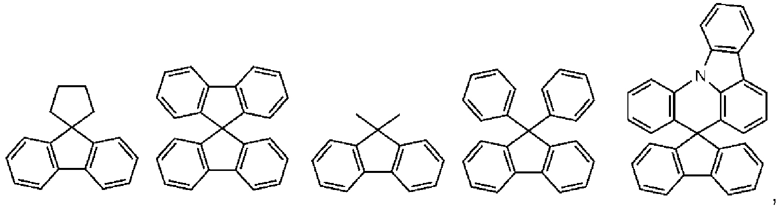 and the like can be formed. However, the structure is not limited thereto.

In the present disclosure, a heterocyclic group is a heterocyclic group containing at least one heteroatom of O, N, Si and S as a heterogeneous element, and the carbon number thereof is not particularly limited, but is preferably 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group, and the arylamine group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine can apply the aforementioned description of the heterocyclic group. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heterocyclic group can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heterocyclic group can be applied, except that the heterocycle is not a monovalent group but formed by combining two substituent groups.

### (Compound)

In the present disclosure, there is provided a compound represented by the following Chemical Formula 1.

Preferably, L₁ is terphenyldiyl, or quaterphenyldiyl.

Preferably, L₁ is any one selected from the group consisting of:

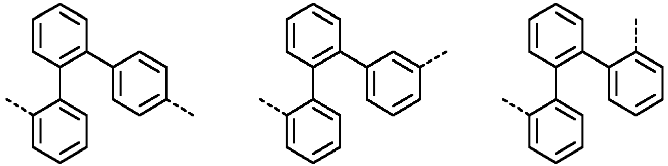

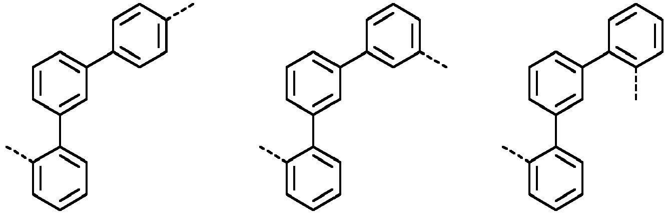

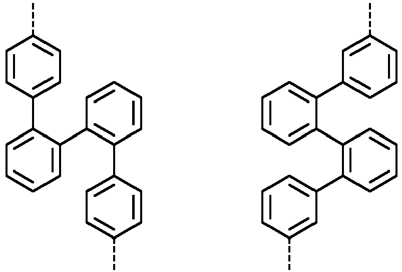

Preferably, at least one of X₁ to X₃ is N; and at least one of X₄ to X₆ is N.

Preferably, L₂ to L₅ are a single bond, or phenylene.

Preferably, Ar₁ to Ar₄ are each independently phenyl, biphenylyl, terphenylyl, or phenylnaphthyl.

Preferably, Ar₁ and Ar₄ are each independently phenyl, biphenylyl, terphenylyl, or phenylnaphthyl; and Ar₂ and Ar₃ are each independently phenyl.

Preferably, at least one of Ar₁ and Ar₄ is substituted with cyano.

Representative examples of the compound represented by the Chemical Formula 1 are as follows:

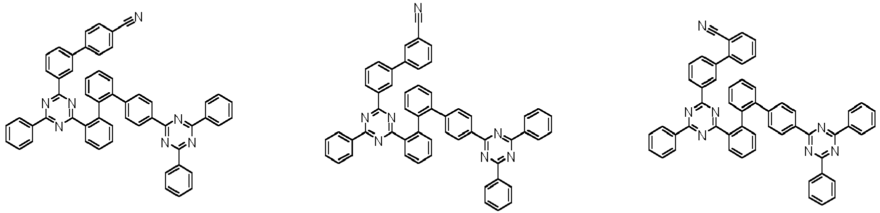

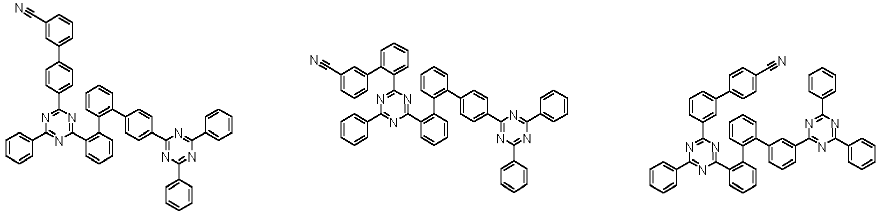

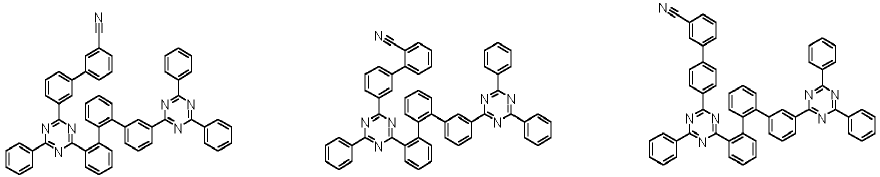

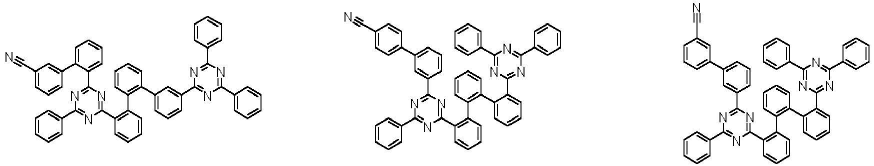

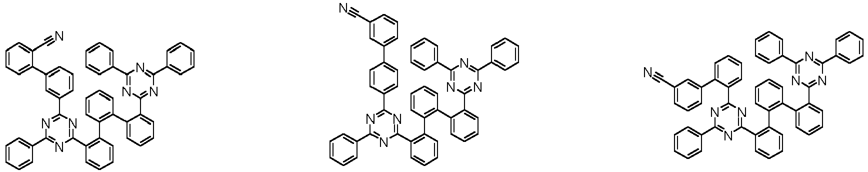

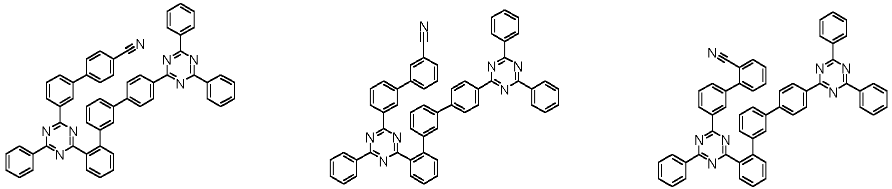

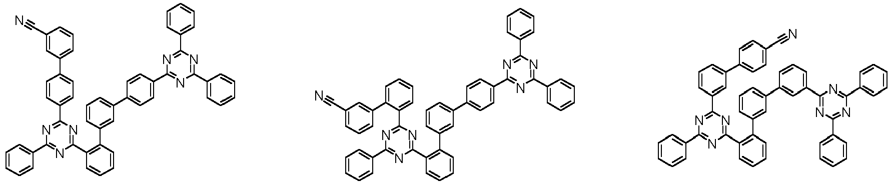

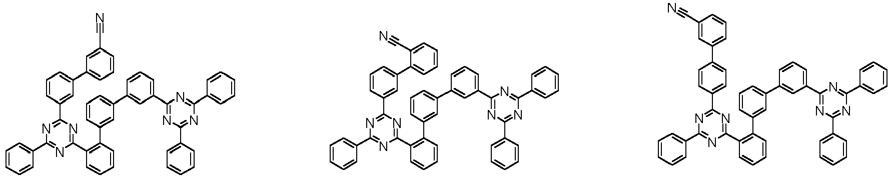

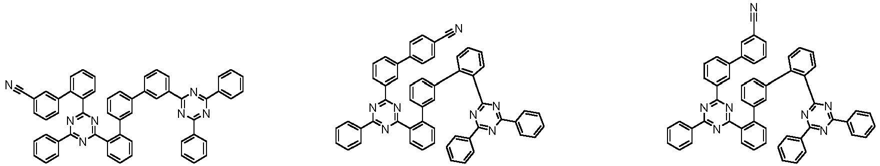

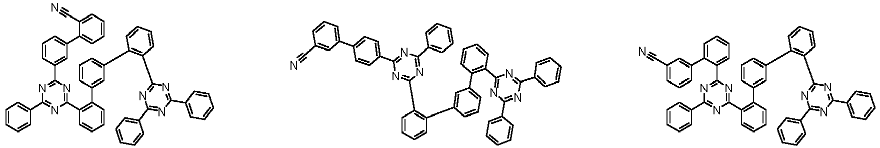

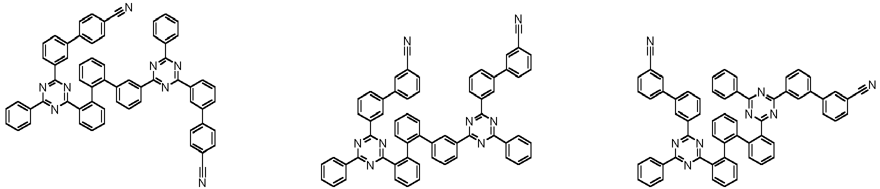

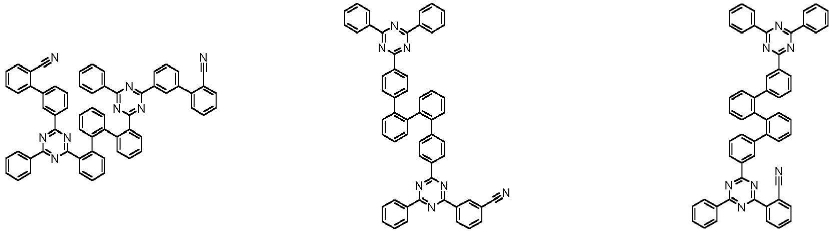

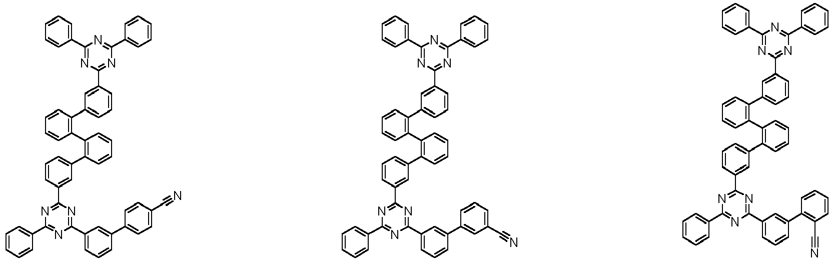

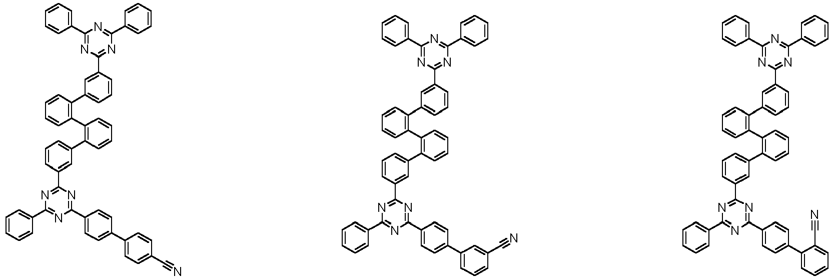

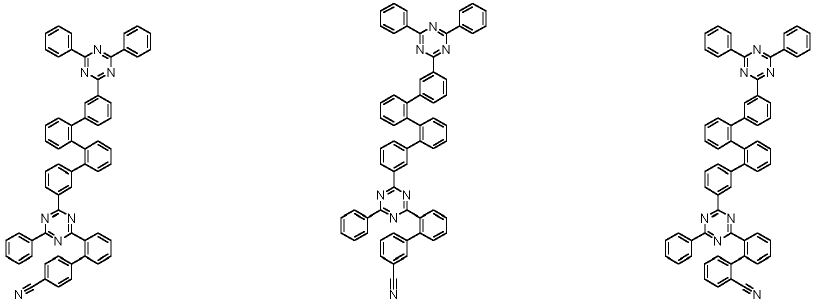

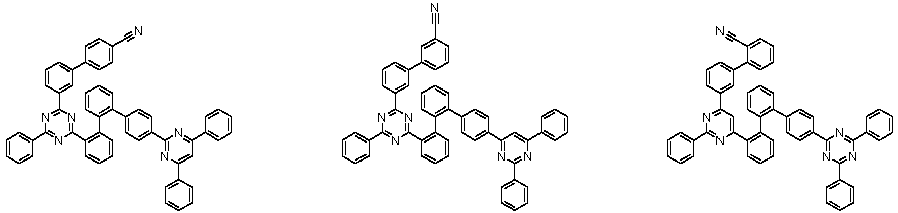

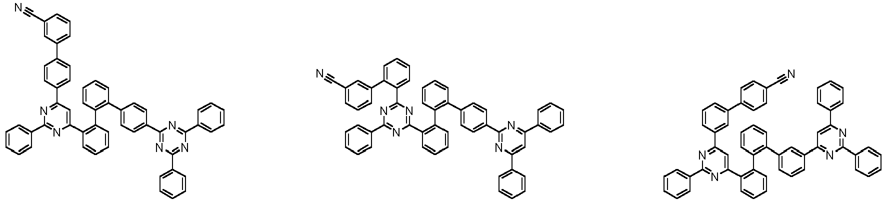

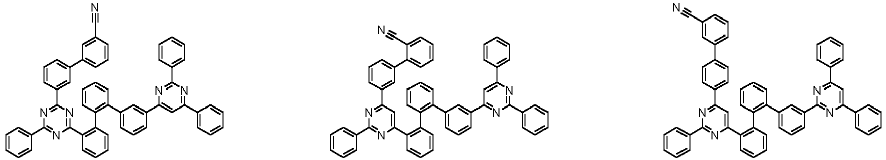

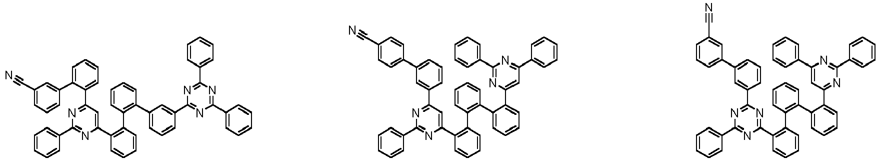

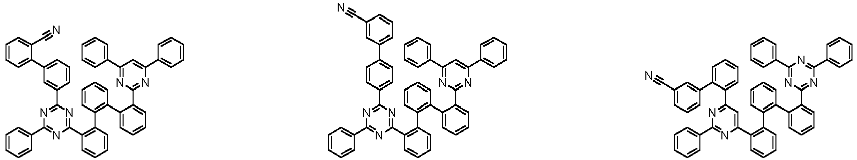

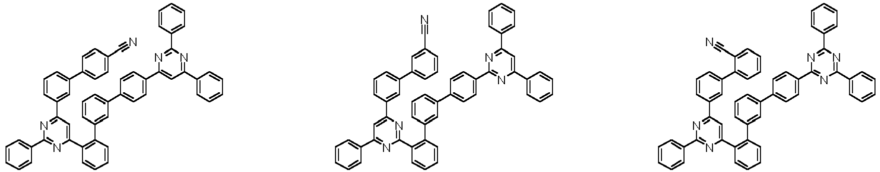

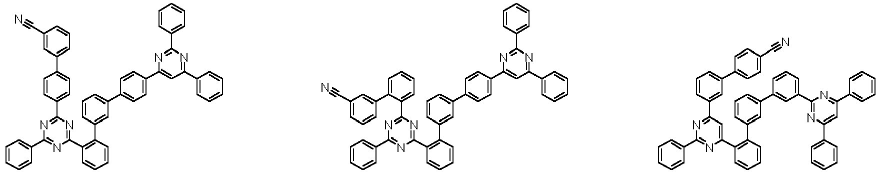

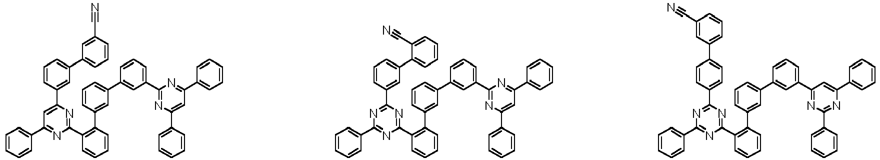

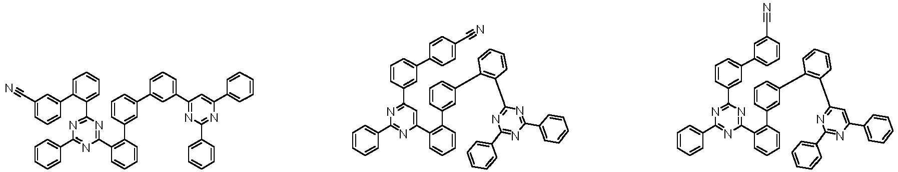

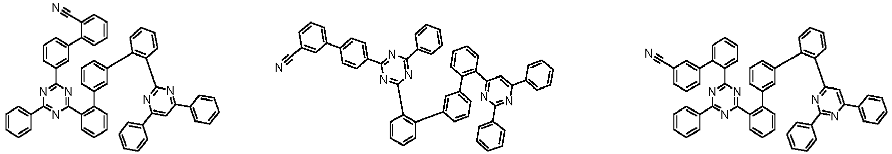

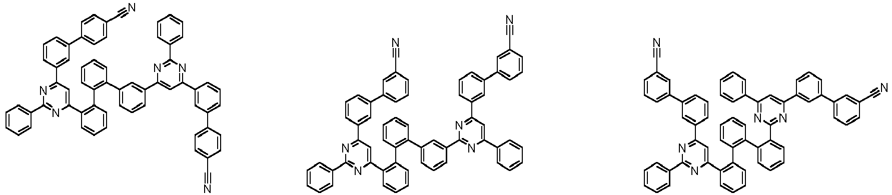

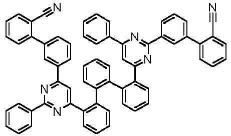

In addition, there is provided a method for preparing a compound represented by the Chemical Formula 1 as shown in Reaction Scheme 1 below.

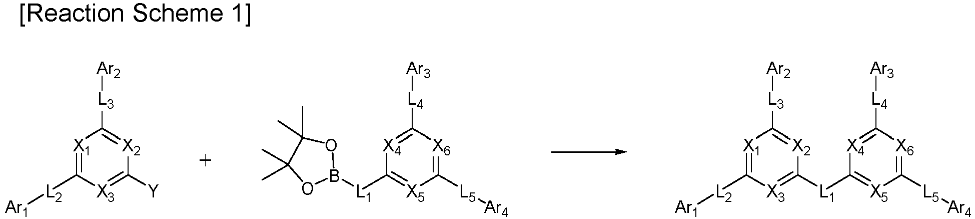

In the Reaction Scheme 1, definitions of other substituents except for Y are the same as defined above, and Y is halogen, preferably bromo, or chloro.

The above reaction is preferably performed in the presence of a palladium catalyst and a base, and the reactive group for the reaction may be appropriately changed. The preparation method may be more specifically described in Preparation Examples described below.

### (Organic light emitting device)

In addition, there is provided an organic light emitting device including the compound represented by the Chemical Formula 1. As an example, there is provided an organic light emitting device including: a first electrode; a second electrode that is provided opposite to the first electrode; and one or more organic material layers that are provided between the first electrode and the second electrode, wherein at least one layer of the organic material layers includes the compound represented by the Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure including a hole injection layer, a hole transport layer, an electron blocking layer, a light emitting layer, an electron transport layer, an electron injection layer, and the like as the organic material layer. However, the structure of the organic light emitting device is not limited thereto, and it may include a smaller number of organic layers.

In addition, the organic material layer may include a hole injection layer, a hole transport layer, or a hole injection and transport layer, and the hole injection layer, the hole transport layer, or the hole injection and transport layer may include the compound represented by the Chemical Formula 1.

In addition, the organic material layer may include a light emitting layer, and the light emitting layer may include the compound represented by the Chemical Formula 1. In particular, the compound according to the present disclosure can be used as a dopant in the light emitting layer.

In addition, the organic material layer may include an electron injection layer, an electron transport layer, or an electron injection and transport layer, and the electron injection layer, the electron transport layer, or the electron injection and transport layer may include the compound represented by the Chemical Formula 1.

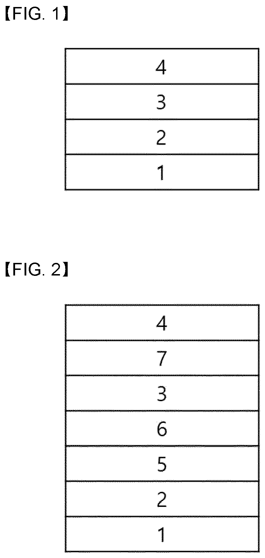

Further, the organic light emitting device according to the present disclosure may be a normal type organic light emitting device in which an anode, one or more organic material layers and a cathode are sequentially stacked on a substrate. Further, the organic light emitting device according to the present disclosure may be an inverted type organic light emitting device in which a cathode, one or more organic material layers and an anode are sequentially stacked on a substrate. For example, the structure of an organic light emitting device according to an embodiment of the present disclosure is illustrated in FIGS. 1 and 2.

FIG. 1 shows an example of an organic light emitting device including a substrate 1, an anode 2, a light emitting layer 3, and a cathode 4. In such a structure, the compound represented by the Chemical Formula 1 may be included in the light emitting layer.

FIG. 2 shows an example of an organic light emitting device including a substrate 1, an anode 2, a hole injection layer 5, a hole transport layer 6, a light emitting layer 3, an electron injection and transport layer 7, and a cathode 4. In such a structure, the compound represented by the Chemical Formula 1 may be included in the electron injection and transport layer.

The organic light emitting device according to the present disclosure may be manufactured using materials and methods known in the art, except that at least one layer of the organic material layers includes the compound represented by the Chemical Formula 1. Moreover, when the organic light emitting device includes a plurality of organic material layers, the organic material layers may be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device may be manufactured by sequentially depositing a cathode material, an organic material layer and an anode material on a substrate.

Further, the compound represented by the Chemical Formula 1 may be formed into an organic material layer by a solution coating method as well as a vacuum deposition method at the time of manufacturing an organic light emitting device. Herein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

For example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole-injecting effect to the light emitting layer or the light emitting material, prevents excitons produced in the light emitting layer from moving to an electron injection layer or the electron injection material, and is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

In addition, the hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The light emitting material is suitably a material capable of emitting light in a visible ray region by receiving holes and electrons from the hole transport layer and the electron transport layer, respectively, to combine them, and having good quantum efficiency to fluorescence or phosphorescence. Specific examples thereof include 8-hydroxy-quinoline aluminum complex (Alq₃); a carbazole-based compound; a dimerized styryl compound; BAlq; a 10-hydroxybenzo quinoline-metal compound; a benzoxazole-, benzthiazole- and benzimidazole-based compound; a poly(p-phenylenevinylene) (PPV)-based polymer; a spiro compound; polyfluorene, rubrene, and the like, but are not limited thereto.

In addition, the light emitting layer may include a host material and a dopant material. The host material may be a fused aromatic ring derivative or a heterocycle-containing compound. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto.

The dopant material includes an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an indium complex, a platinum complex, and the like, but is not limited thereto.

The electron transport layer is a layer which receives electrons from an electron injection layer and transports the electrons to a light emitting layer, and an electron transport material used is suitably a material which can receive electrons well from a cathode and transfer the electrons to a light emitting layer and has large mobility for electrons. Specifically, examples thereof may include an Al complex of 8-hydroxyquinoline; a complex including Alq₃; an organic radical compound; a hydroxyflavone-metal complex, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

The organic light emitting device according to the present disclosure may be a front side emission type, a backside emission type, or a double-sided emission type according to the used material.

In addition, the compound according to the present disclosure may be included in an organic solar cell or an organic transistor in addition to an organic light emitting device.

The preparation of the compound represented by the Chemical Formula 1 and the organic light emitting device including the same will be described in detail in the following examples. However, these examples are presented for illustrative purposes only, and are not intended to limit the scope of the present disclosure.

### [Examples]

### Example 1: Preparation of Compound E1

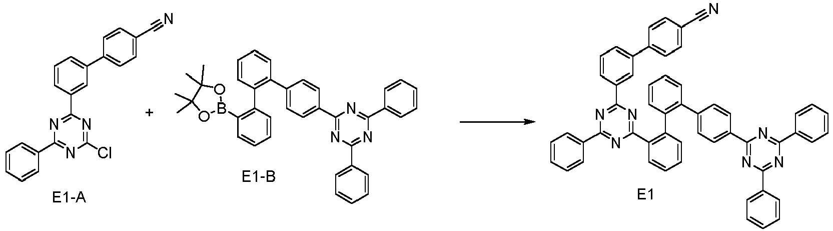

E1-A (20 g, 54.2 mmol) and E1-B (31.9 g, 54.2 mmol) were added to tetrahydrofuran (400 ml) under a nitrogen atmosphere, and the mixture was stirred and refluxed. Then, potassium carbonate (22.5 g, 162.7 mmol) was dissolved in water (22 ml), and then added thereto. Thereafter, it was stirred sufficiently, followed by adding tetrakistriphenyl-phosphinopalladium (1.9 g, 1.6 mmol). After 3 hours of reaction, cooling was performed to room temperature. Then, the organic layer was separated from the water layer, and then the organic layer was distilled. Then, this was dissolved again in chloroform (20 times, 861 mL), and washed twice with water. Thereafter, the organic layer was separated, treated with anhydrous magnesium sulfate, stirred, then filtered, and the filtrate was distilled under reduced pressure. The concentrated compound was recrystallized with chloroform and ethyl acetate to prepare Compound E1 in the form of white solid (22.8 g, 53%).
MS: [M+H]⁺ = 794

### Example 2: Preparation of Compound E2

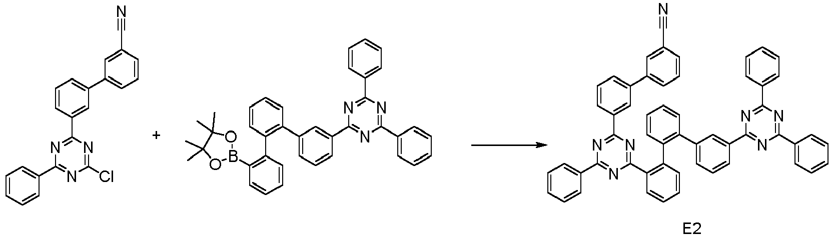

Compound E2 was prepared in the same manner as in Example 1, except that each starting material was used as in the above reaction scheme.
MS: [M+H]⁺ = 794

### Example 3: Preparation of Compound E3

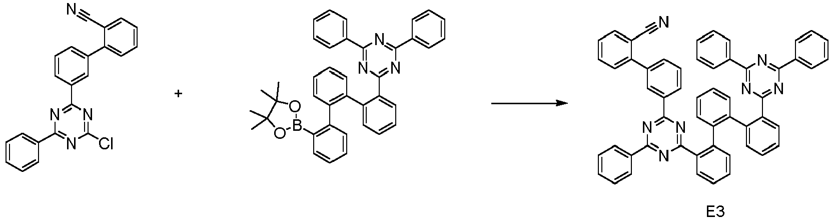

Compound E3 was prepared in the same manner as in Example 1, except that each starting material was used as in the above reaction scheme.
MS: [M+H]⁺ = 794

### Example 4: Preparation of Compound E4

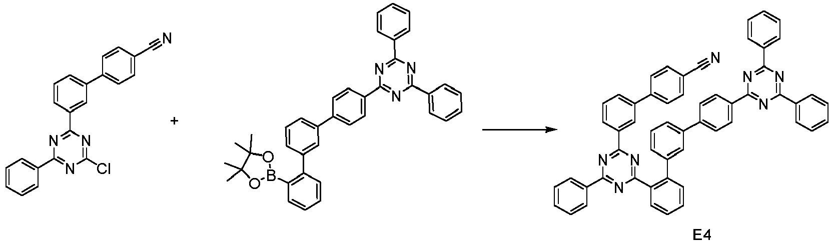

Compound E4 was prepared in the same manner as in Example 1, except that each starting material was used as in the above reaction scheme.
MS: [M+H]⁺ = 794

### Example 5: Preparation of Compound E5

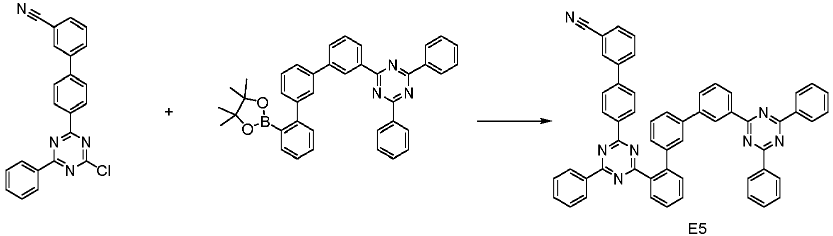

Compound E5 was prepared in the same manner as in Example 1, except that each starting material was used as in the above reaction scheme.
MS: [M+H]⁺ = 794

### Example 6: Preparation of Compound E6

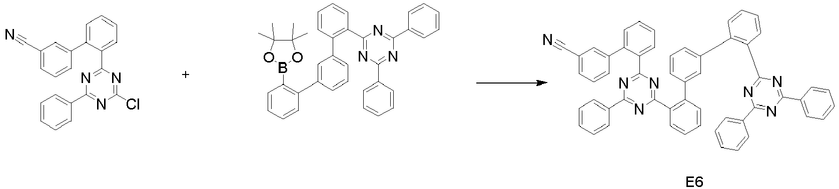

Compound E6 was prepared in the same manner as in Example 1, except that each starting material was used as in the above reaction scheme.
MS: [M+H]⁺ = 794

### Example 7: Preparation of Compound E7

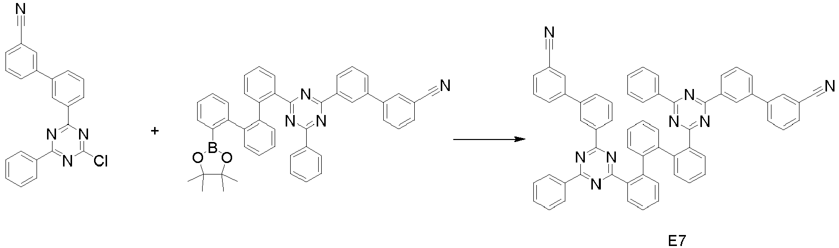

Compound E7 was prepared in the same manner as in Example 1, except that each starting material was used as in the above reaction scheme.
MS: [M+H]⁺ = 895

### Example 8: Preparation of Compound E8

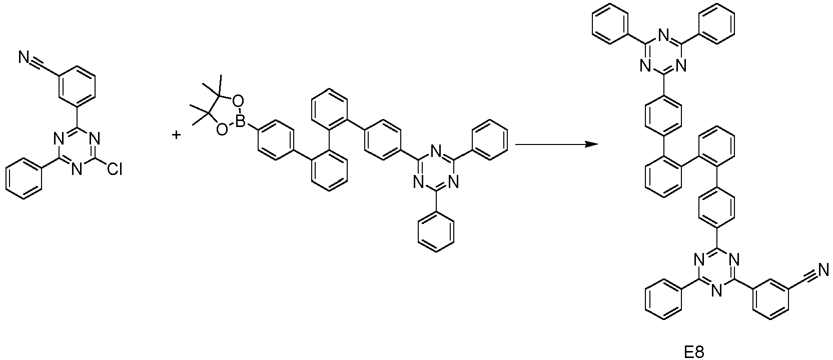

Compound E8 was prepared in the same manner as in Example 1, except that each starting material was used as in the above reaction scheme.
MS: [M+H]⁺ = 794

### Example 9: Preparation of Compound E9

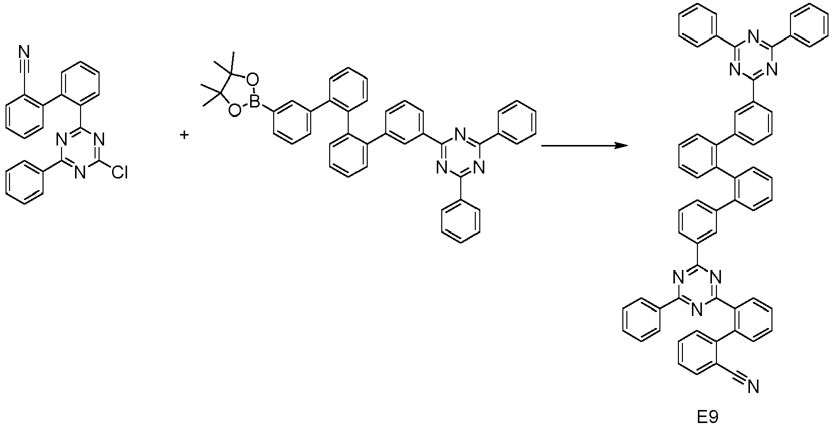

Compound E9 was prepared in the same manner as in Example 1, except that each starting material was used as in the above reaction scheme.
MS: [M+H]⁺ = 870

### Example 10: Preparation of Compound E10

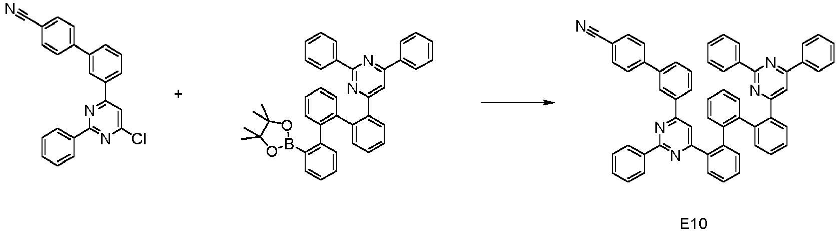

Compound E10 was prepared in the same manner as in Example 1, except that each starting material was used as in the above reaction scheme.
MS: [M+H]⁺ = 792

### Example 11: Preparation of Compound E11

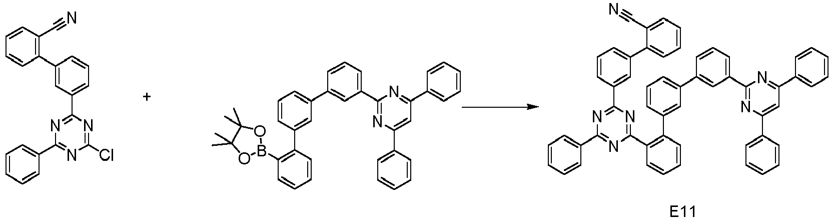

Compound E11 was prepared in the same manner as in Example 1, except that each starting material was used as in the above reaction scheme.
MS: [M+H]⁺ = 793

### Example 12: Preparation of Compound E12

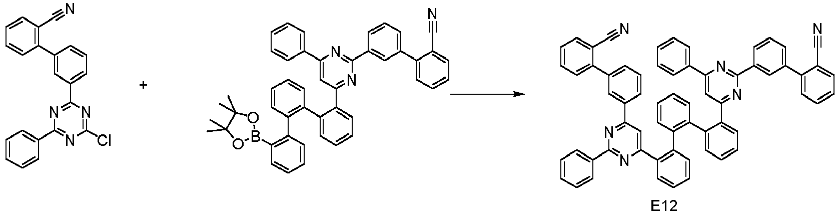

Compound E12 was prepared in the same manner as in Example 1, except that each starting material was used as in the above reaction scheme.
MS: [M+H]⁺ = 893

### Example 13: Preparation of Compound E13

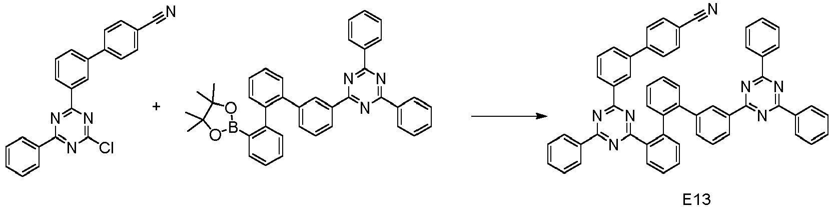

Compound E13 was prepared in the same manner as in Example 1, except that each starting material was used as in the above reaction scheme.
MS: [M+H]⁺ = 794

### [Experimental Examples]

### Experimental Example 1

A glass substrate on which ITO (indium tin oxide) was coated as a thin film to a thickness of 1,000 Å was put into distilled water in which a detergent was dissolved, and ultrasonically cleaned. At this time, a product manufactured by Fischer Co. was used as the detergent, and distilled water filtered twice using a filter manufactured by Millipore Co. was used as the distilled water. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol, dried, and then transferred to a plasma cleaner. In addition, the substrate was cleaned for 5 minutes using oxygen plasma and then transferred to a vacuum depositor.

On the prepared ITO transparent electrode, the following Compound HI-A was thermally vacuum-deposited to a thickness of 600 Å to form a hole injection layer. On the hole injection layer, the following Compound HAT (50 Å) and the following Compound HT-A (60 Å) were sequentially vacuum-deposited to form a first hole transport layer and a second hole transport layer.

Then, the following Compounds BH and BD were vacuum-deposited on the second hole transport layer at a weight ratio of 25:1 to a thickness of 200 Å to form a light emitting layer.

The prepared Compound E1 and the following Compound LiQ were vacuum-deposited on the light emitting layer at a weight ratio of 1:1 to a thickness of 350 Å to form an electron injection and transport layer. On the electron injection and transport layer, lithium fluoride (LiF) and aluminum were sequentially deposited to a thickness of 10 Å and 1,000 Å, respectively to form a cathode.

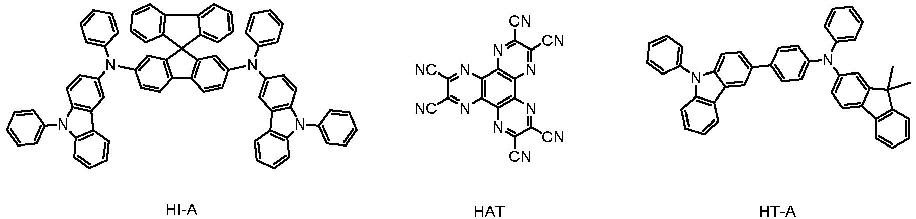

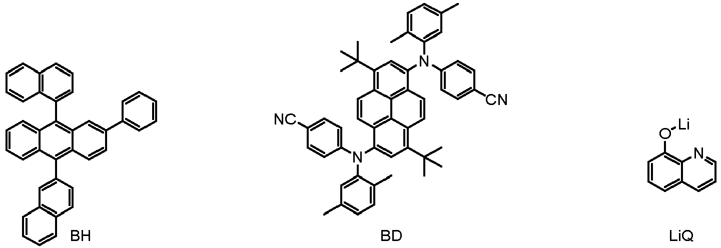

In the above process, the deposition rate of the organic material was maintained at 0.4 to 0.9 Å/sec, the deposition rate of lithium fluoride of the cathode was maintained at 0.3 Å/sec, and the deposition rate of aluminum was maintained at 2 Å/sec. In addition, the degree of vacuum during the deposition was maintained at 1 × 10⁻⁷ torr to 5 × 10⁻⁵ torr, thereby manufacturing an organic light emitting device.

### Experimental Examples 2 to 13

An organic light emitting device was manufactured in the same manner as in Experimental Example 1, except that the compound shown in Table 1 was used instead of the Compound E1.

### Comparative Experimental Examples 1 to 10

An organic light emitting device was manufactured in the same manner as in Experimental Example 1, except that the compound shown in Table 1 was used instead of the Compound E1. At this time, Compounds ET-A to ET-J listed in Table 1 are as follows.

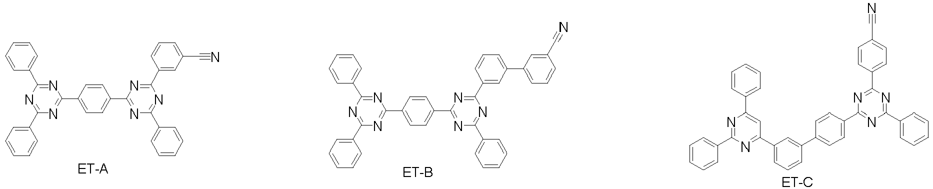

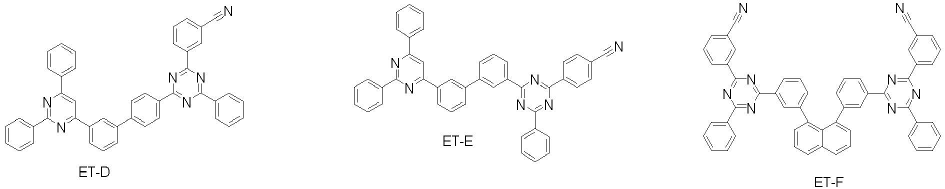

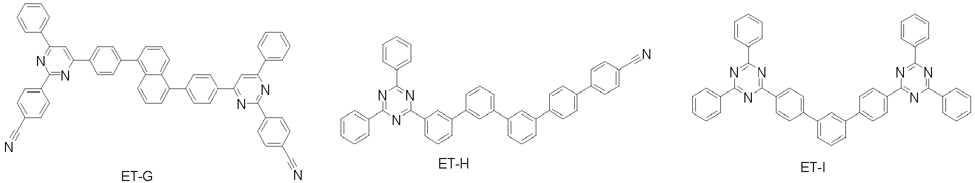

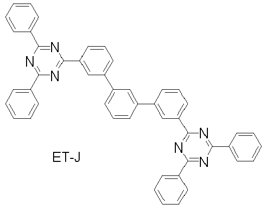

For the organic light emitting devices prepared in Experimental Examples 1 to 12 and Comparative Experimental Examples 1 to 10, the driving voltage and luminous efficiency were measured at a current density of 10 mA/cm². In addition, T₉₀, which is the time taken until the initial luminance decreases to 90% at a current density of 20 mA/cm², was measured. The results are shown in Table 1 below.

**[Table 1]**

| Category | Compound | Driving voltage (V@10mA/cm²) | Efficiency (cd/A@10mA/cm²) | Chromaticity coordinates (x, y) | Lifespan (hr) (T90@20mA/cm²) |
|---|---|---|---|---|---|
| Experimental Example 1 | E1 | 4.05 | 5.38 | (0.140, 0.093) | 246 |
| Experimental Example 2 | E2 | 4.17 | 5.27 | (0.140, 0.095) | 271 |
| Experimental Example 3 | E3 | 4.09 | 5.43 | (0.140, 0.094) | 231 |
| Experimental Example 4 | E4 | 3.97 | 5.54 | (0.141, 0.093) | 207 |
| Experimental Example 5 | E5 | 4.21 | 5.16 | (0.140, 0.093) | 308 |
| Experimental Example 6 | E6 | 4.09 | 5.33 | (0.140, 0.093) | 251 |
| Experimental Example 7 | E7 | 4.29 | 5.06 | (0.140, 0.093) | 384 |
| Experimental Example 8 | E8 | 4.14 | 5.27 | (0.140, 0.095) | 280 |
| Experimental Example 9 | E9 | 4.09 | 5.48 | (0.140, 0.094) | 224 |
| Experimental Example 10 | E10 | 4.33 | 5.00 | (0.141, 0.093) | 221 |
| Experimental Example 11 | E11 | 4.29 | 5.06 | (0.140, 0.093) | 249 |
| Experimental Example 12 | E12 | 4.37 | 4.90 | (0.140, 0.093) | 241 |
| Experimental Example 13 | E13 | 4.06 | 5.40 | (0.140, 0.093) | 260 |
| Comparative Experimental Example 1 | ET-A | 4.90 | 2.69 | (0.140, 0.095) | 74 |
| Comparative Experimental Example 2 | ET-B | 4.80 | 2.74 | (0.140, 0.094) | 59 |
| Comparative Experimental Example 3 | ET-C | 5.05 | 2.58 | (0.141, 0.093) | 81 |
| Comparative Experimental Example 4 | ET-D | 5.00 | 2.61 | (0.140, 0.093) | 77 |
| Comparative Experimental Example 5 | ET-E | 4.90 | 2.58 | (0.140, 0.093) | 54 |
| Comparative Experimental Example 6 | ET-F | 4.85 | 2.80 | (0.140, 0.093) | 185 |
| Comparative Experimental Example 7 | ET-G | 4.95 | 2.74 | (0.140, 0.095) | 166 |
| Comparative Experimental Example 8 | ET-H | 4.46 | 3.77 | (0.140, 0.094) | 148 |
| Comparative Experimental Example 9 | ET-I | 4.25 | 4.84 | (0.141, 0.093) | 25 |
| Comparative Experimental Example 10 | ET-J | 4.21 | 4.79 | (0.140, 0.093) | 20 |

As shown in Table 1, the compound represented by the Chemical Formula 1 of the present disclosure may be used in an organic material layer capable of simultaneously performing electron injection and electron transport of an organic light emitting device.

As a result of comparing Experimental Examples 1 to 13 and Comparative Experimental Examples 1 to 5 of Table 1, it was confirmed that the organic light emitting device including the compound represented by the Chemical Formula 1 of the present disclosure had significantly superior driving voltage, efficiency and lifespan than the organic light emitting device including a compound in which L1 is phenylene or biphenyldiyl.

As a result of comparing Experimental Examples 1 to 13 and Comparative Experimental Examples 6 and 7 of Table 1, it was confirmed that the organic light emitting device including the compound represented by the Chemical Formula 1 of the present disclosure had significantly superior driving voltage, efficiency and lifespan than the organic light emitting device including a compound in which L1 is naphthylene.

As a result of comparing Experimental Examples 1 to 13 and Comparative Experimental Examples 9 and 10 of Table 1, it was confirmed that the organic light emitting device including the compound represented by the Chemical Formula 1 of the present disclosure had significantly superior lifespan than the organic light emitting device including a cyano-unsubstituted compound.

**[DESCRIPTION OF SYMBOLS]**

| | | | |
|---|---|---|---|
| 1: | Substrate | 2: | Anode |
| 3: | Light emitting layer | 4: | Cathode |
| 5: | Hole injection layer | 6: | Hole transport layer |
| 7: | Electron injection and transport layer | | |

## Claims

1. A compound represented by the following Chemical Formula 1: 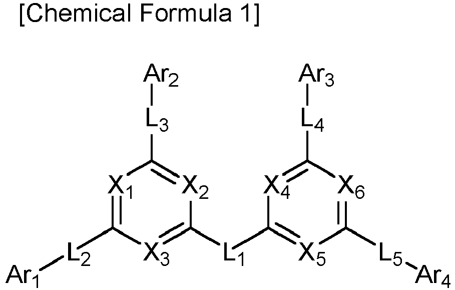 in the Chemical Formula 1,
X₁ to X₆ are each independently N or CH, wherein at least two of X₁ to X₆ are N,
L₁ is represented by the following Chemical Formula 2,
L₂ to L₅ are each independently a single bond, or substituted or unsubstituted C6-60 arylene,
Ar₁ to Ar₄ are each independently substituted or unsubstituted C₆₋₆₀ aryl, and
at least one of Ar₁ to Ar₄ is substituted with cyano, 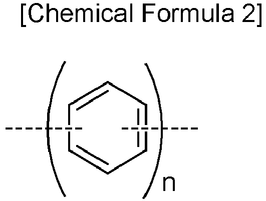
in the Chemical Formula 2,
n is an integer of 3 to 5.

2. The compound of Claim 1,
wherein L₁ is terphenyldiyl, or quaterphenyldiyl.

3. The compound of Claim 1,
wherein L₁ is any one selected from the group consisting of:

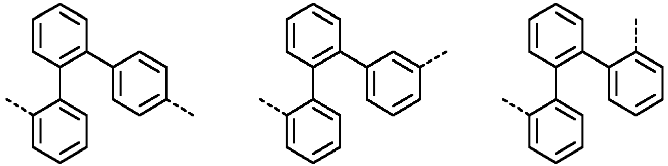

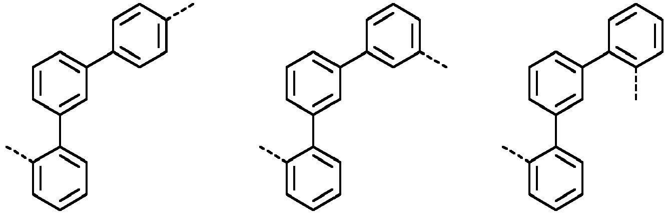

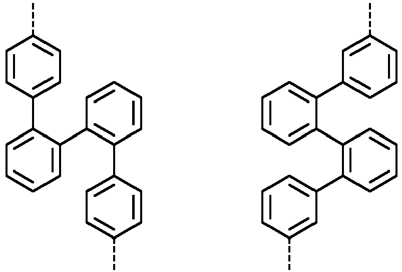

4. The compound of Claim 1,
wherein at least one of X₁ to X₃ is N, and
at least one of X₄ to X₆ is N.

5. The compound of Claim 1,
wherein L₂ to L₅ are a single bond, or phenylene.

6. The compound of Claim 1,
wherein Ar₁ to Ar₄ are each independently phenyl, biphenylyl, terphenylyl, or phenylnaphthyl.

7. The compound of Claim 1,
wherein Ar₁ and Ar₄ are each independently phenyl, biphenylyl, terphenylyl, or phenylnaphthyl, and
Ar₂ and Ar₃ are each independently phenyl.

8. The compound of Claim 1,
wherein at least one of Ar₁ and Ar₄ is substituted with cyano.

9. The compound of Claim 1,
wherein the compound represented by the Chemical Formula 1 is any one selected from the group consisting of the following compounds:

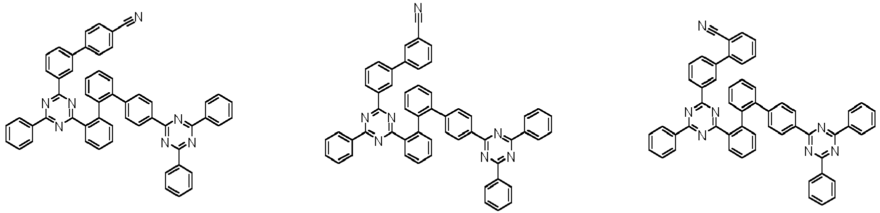

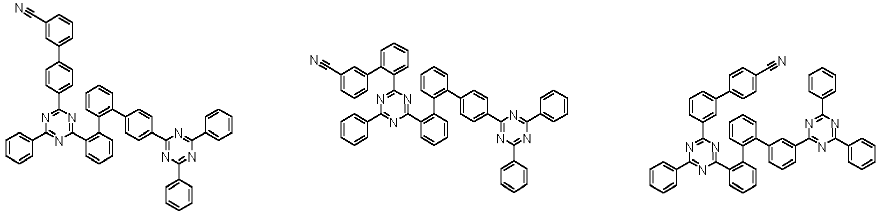

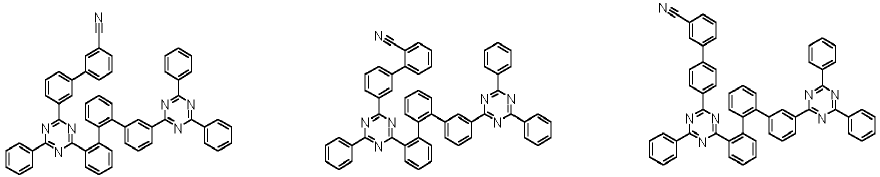

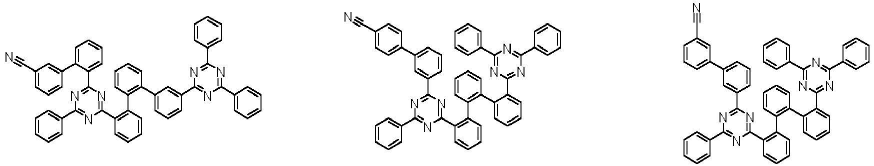

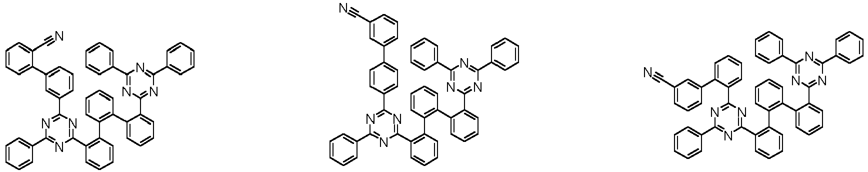

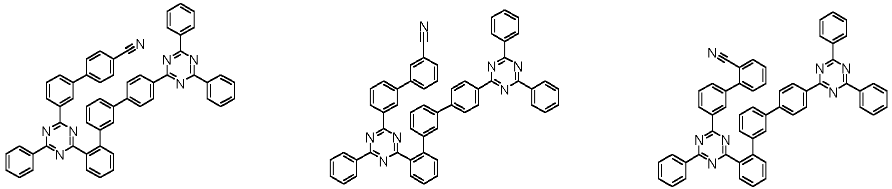

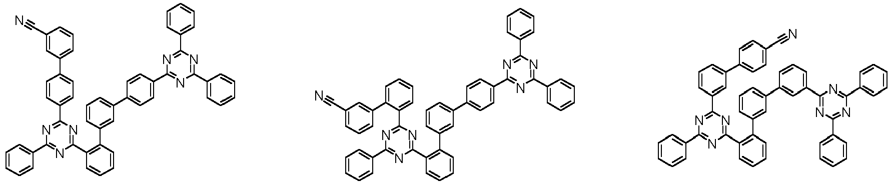

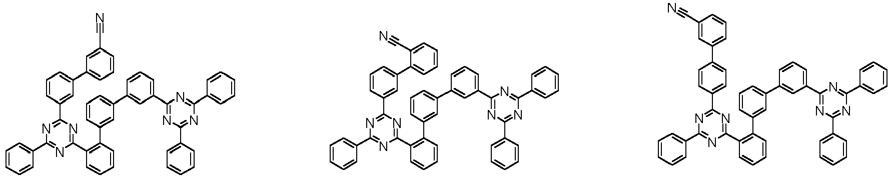

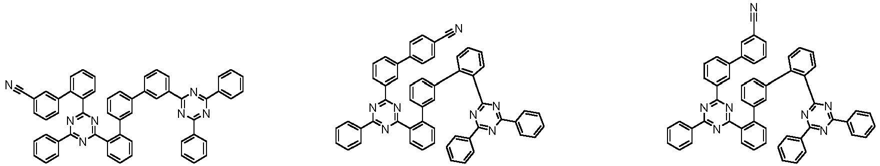

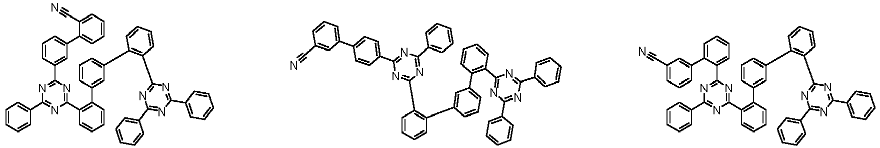

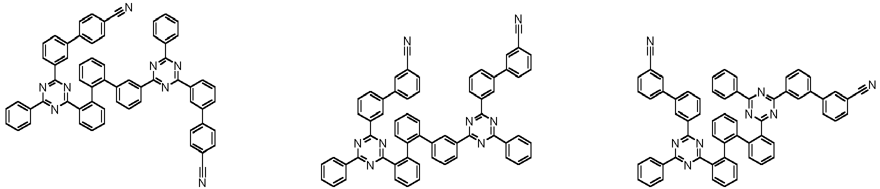

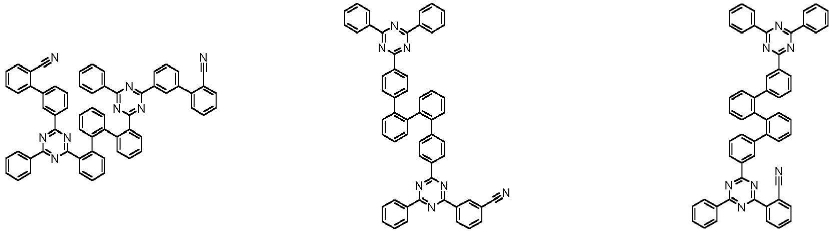

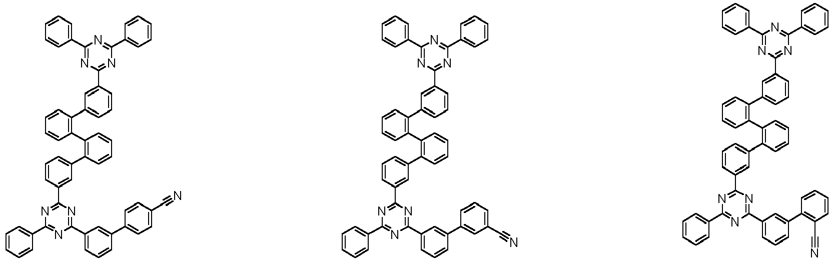

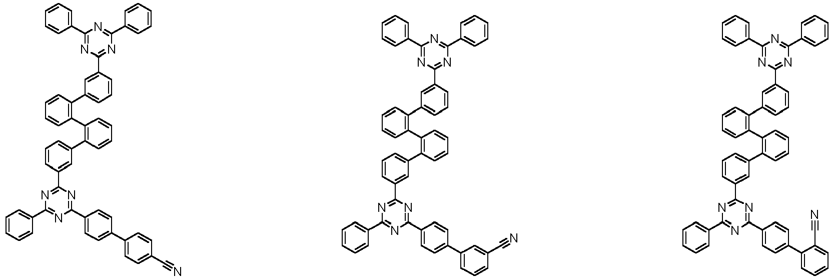

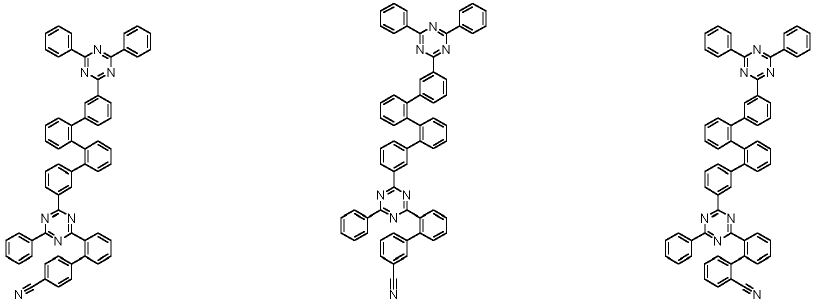

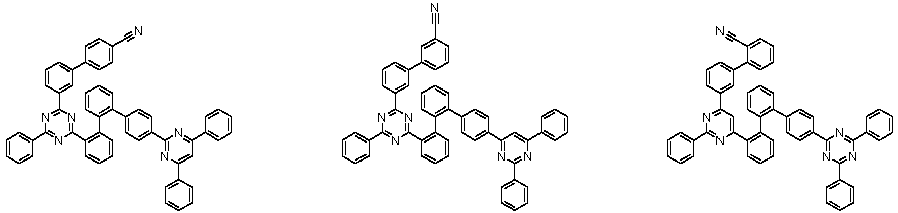

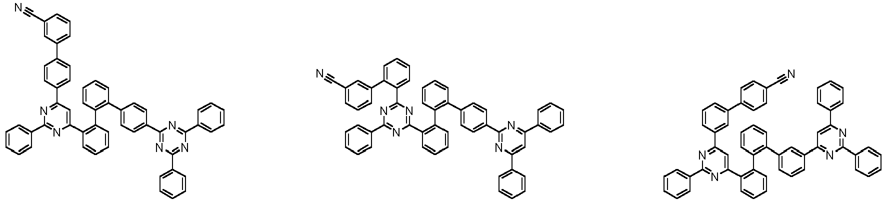

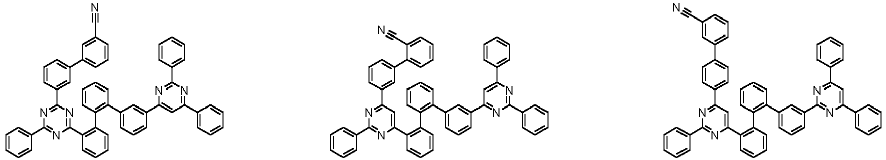

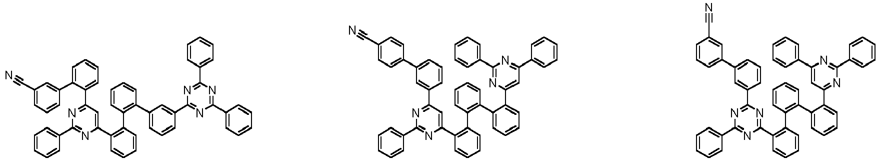

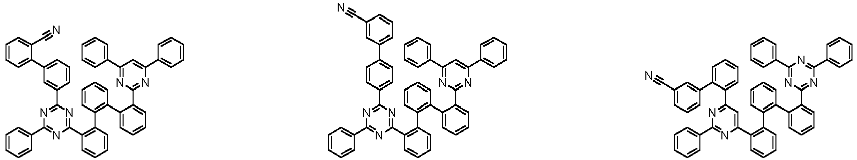

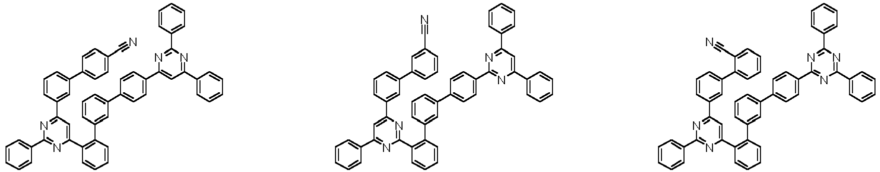

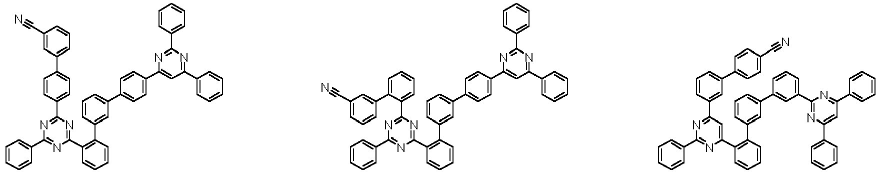

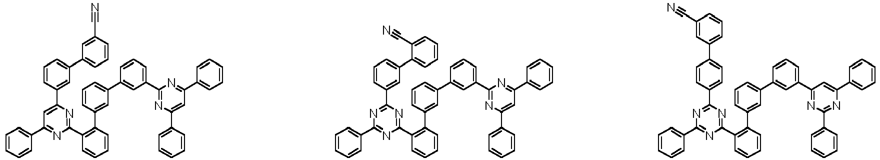

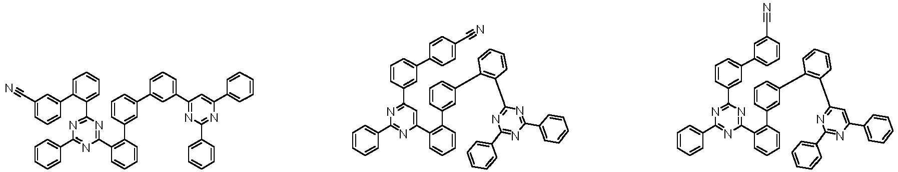

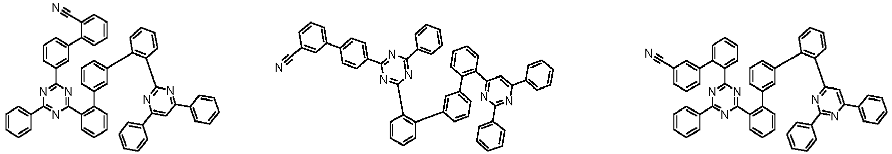

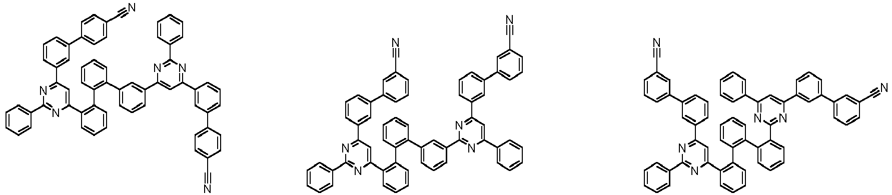

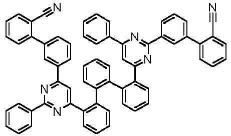

10. An organic light emitting device comprising: a first electrode; a second electrode that is provided opposite to the first electrode; and one or more organic material layers that are provided between the first electrode and the second electrode, wherein at least one layer of the organic material layers comprises the compound according to any one of Claims 1 to 9.

11. The organic light emitting device of Claim 10,
wherein the organic material layer is an electron injection layer, an electron transport layer, or an electron injection and transport layer.
